# EUROPEAN PATENT SPECIFICATION

(11) **EP 0 338 807 B1**
(45) Date of publication and mention of the grant of the patent: **02.11.1995**
(21) Application number: 89303887.7
(22) Date of filing: 18.04.1989
(51) Int. Cl.: C12N 15/86, C12N 5/00

(54) **Production of isolated proteinaceous materials using recombinant avipox virus vectors**
Herstellung von isoliertem proteinartigem Material unter Anwendung von rekombinanten Avipox-Virus-Vektoren
Production de matériaux protéiniques isolés en utilisant des vecteurs de virus avipox recombinés

(30) Priority: 19.04.1988 DE 3813093
(43) Date of publication of application: 25.10.1989
(73) Proprietor: IMMUNO Aktiengesellschaft, A-1221 Wien (AT)
(72) Inventor: Falkner, Falko Günther, Dr., A-2304 Mannsdorf 116 (AT); Bodemer, Walter, A-1228 Wien (AT); Dorner, Friedrich, Prof., A-1230 Wien (AT)
(74) Representative: Hansen, Bernd, Dr. Dipl.-Chem.

(56) References cited:
- EP-A- 0 162 782
- EP-A- 0 284 416
- EP-A- 0 308 220
- WO-A-86/05806
- WO-A-88/02022
- WO-A-89/03429
- WO-A-89/12684

## Description

The invention relates to the expression in animal tissue culture of foreign DNA, particularly DNA coding for viral glycoproteins and vitamin K-dependent blood factors, in which the foreign DNA is expressed in an avipox virus expression vector.

By virtue of the development of modern genetic methods it has been possible to identify and isolate a plurality of genes for prophylactic, diagnostic and therapeutically significant proteins, eg the genes for blood clotting factors or antigenic virus proteins. In this connection the need for reliable and efficient expression systems in prokaryotic or eukaryotic cells is increasing in order to be able to isolate the desired proteins either from infected tissue or infected cell culture or eg to make use of the immunogenic properties in vivo of expressed proteins for immunisation. Eukaryotic viral vector systems have thereby acquired a particular significance in this connection.

WO-A-8802022 and WO-A-86/05806 discloses the use of recombinant fowlpox virus in the preparation of fowlpox vaccines and other delivery systems for poultry. The question of whether recombinant avipox viruses would make suitable expression vectors for the production of isolated proteinaceous materials is not addressed.

Various prior viral expression vector systems for use in the preparation of isolated proteinaceous materials have however been proposed. One of the known eukaryotic vector systems is the Papova-Virus SV40. With 5.4kb SV40 has a comparatively small genome whose organisation is relatively well known. The small genome of this virus has on the one hand the advantage that it can be precisely manipulated when using suitable restriction endonucleases but on the other hand the disadvantage that the cloning capacity is severely limited by a correspondingly small virus shell and by the dense, partially overlapping arrangement of essential genes.

The development of a eukaryotic vector system on the basis of vaccinia virus has also been described (eg EP-A-0243029). The vaccinia virus is an orthopox virus with a linear genome of double stranded DNA of about 185 kbp. The size of the vaccinia virus genome renders impossible the construction of recombinant genomes by cleavage with restriction endonucleases and subsequently ligation with suitable foreign DNA. Even if this method were usable, the production of viruses with the recombinant genome would require the presence of a helper virus since the in vitro manipulated virus DNA is itself not infectious. The transcription of the vaccinia virus requires inter alia that a vaccinia virus coded RNA polymerase, which cannot be substituted by the eukaryotic RNA polymerase II, must be brought in packaged in the virus particle during the infection. Accordingly the vaccinia virus has special promoters tailored to the virus coded RNA polymerase. This means further difficulty in the construction of recombinants which are to express the foreign genes inserted into the vaccinia virus genome in eukaryotic cells. The use of vaccinia viruses as expression vectors thus requires that the transcription and replication can be performed in a host-independent manner by vaccinia-specific enzymes, ie that on the one hand no essential virus regions may be effected by the cloning and that on the other hand genes provided for expression must be under the control of vaccinia promoters.

For this reason a method including two steps was used in earlier work in which firstly a plasmid was constructed which contained the desired foreign gene in conjunction with a vaccinia virus promoter and this recombinant plasmid was introduced into the vaccinia virus genome by homologous recombination between virus and plasmid-DNA in vivo. For instance, in EP-A-0243029 a recombinant vaccinia virus is described which was constructed in accordance with the principle described above and is used for expressing the HIV env-Protein.

However, the use of vaccinia virus vectors has the substantial disadvantage that the virus which is pathogenic for very many species is also pathogenic for humans. In order to protect personnel, and also when removing waste, costly safety measures must therefore be taken. These safety measures are both time consuming and very expensive. It would therefore be desirable to use a vector system for expression in eukaryotic cells which on the one hand has the advantages of a vaccinia system but on the other hand may be handled more easily.

It is thus an object of the invention to make available an expression system for foreign DNA, in which the system is not pathogenic for humans.

Subject matter of the present invention is a plasmid comprising a foreign DNA sequence coding for a proteinaceous material according to Claim 1.

Preferred embodiments thereof are subject matter of Claims 2 to 11.

A further subject matter is a recombinant avipox virus expression vector according to Claims 12 and 13.

A preferred embodiment of these virus expression vectors is subject matter of Claim 14.

Further subject matter is a mammalian cell culture and a primary chicken embryo fibroblast cell culture according to Claims 15 and 16, respectively.

A still further subject matter is a process for the preparation of a proteinaceous material according to Claim 17.

Preferred embodiments of this process are subject matter of Claims 18 to 20.

In accordance with one aspect of the invention, there is provided a process for the preparation of a proteinaceous material, the method comprising growing animal cells containing a recombinant avipox virus expression vector expressing foreign DNA coding for the proteinaceous material and harvesting the proteinaceous material.

The expression vector may contain, in addition to natural DNA of avipox virus, an additional promotor which controls the expression of the foreign DNA.

The recombinant avipox virus expression vector can be produced by recombining an avipox virus with a recombinant plasmid including at least one replicon (ori1), at least one selection marker (sm1), at least one cloning site (cs), and at least one promoter (pr1), in which the promoter and the cloning sites are flanked by DNA sequences of an avipox virus. Foreign DNA can be inserted in the cloning site, or into one of the cloning sites.

The use of avipox viruses for establishing an expression system in eukaryotic cells has considerable advantages in comparison to that of vaccinia viruses. Whilst the host range of vaccinia virus extends to humans, avipox viruses are only infectious for birds and cell lines derived from birds. It follows from this that laboratory personnel are not subjected to danger of illness with the use of avipox virus-derived expression systems.

Recombinant plasmids which may be used to prepare recombinant avipox virus expression vectors for use in the invention generally would include in addition to their usual components, which are inevitably necessary for its construction such as a replicon (ori1), at least one selection marker (sm1), cloning sites (cs) and at least one promoter (pr1), DNA sequences from an avipox virus genome, which flank the promoter and the cloning sites. By in vivo recombination with intact avipox virus genome, the insertion of the promoter, the cloning sites and possibly foreign DNA inserted in the cloning sites into the avipox virus genome can be effected by virtue of this flanking arrangement.

Commonly a replicon which enables the replication of the plasmid in prokaryotes is used as the [oril.] For the construction of the recombinant plasmid it is preferred that transformation and amplification of the recombinant plasmid is performed in gram negative bacteria, eg in Escherichia coli. A particularly preferred replicon is thus the Col E 1 replicon from Escherichia coli.

Certain steps in the construction of plasmids useful in the invention, for instance the selection of transformed bacteria, are facilitated by the use of a suitable selection marker. A preferred selection marker when transforming prokaryotes is eg the inactivation by antibiotics. Those skilled in the art will be familiar with a number of suitable resistant genes for the expression of antibiotic inactivating proteins in gram-negative and gram-positive organisms.

Plasmids which can be used in the construction of avipox virus expression vectors useful in the invention contain at least one cloning site (cs), but preferably a multiple cloning site (mcs), which renders possible the insertion of foreign genes using a wide variety of restriction endonucleases. Such mcs's are preferably similarly constructed to the sequences used in the pUC plasmids or in the M13 vectors. These mcs's contain cloning sites for a plurality of conventional restriction endonucleases which generally recognise palindromes of 4 or 6 base pairs.

The cloning site may comprise a sequence which contains the restriction endonuclease recognition sites for the enzymes EcoRI, PstI, SalI, AccI, HincII, XbaI and BamHI. In order to be able to effect cloning in a number of possible reading frames, the cloning site at its 5' end can be modified in different embodiments by insertion of one or two additional bases. This enables expression of the inserted foreign DNA in all three frames. Examples of such sequences are eg the following:
a) ATG AAT TCC TGC AGG TCG ACT CTA GAG GAT CCC CTT AAG TTA ACT TAA;
b) ATG GAA TTC CTG CAG GTC GAC TCT AGA GGA TCC CCT TAA GTT AAC TTA A;
c) ATG GGA ATT CCT GCA GGT CGA CTC TAG AGG ATC CCC TTA AGT TAA CTT AA.

The promoter pr1 which may be contained in the recombinant plasmid is so arranged upstream of the cloning site that it regulates the transcription of the foreign gene to be inserted. Since the avipox virus does not penetrate into the nucleus of the eukaryotic host cell, but is replicated in the cytoplasm like all pox viruses and its transcription is thus dependent on avipox-coded enzymes, a pox virus promoter is conveniently used as promoter for the plasmid which is to be subsequently used for homologous recombination with avipox virus.

In order to achieve as efficient as possible an expression of an inserted foreign gene, a strong promoter can be used as the promoter pr1. Furthermore, this promoter should be active if possible during the entire period of the infection cycle of the avipox virus.

In a preferred embodiment the promoter used originates from the vaccinia virus. The vaccinia virus promoters are the best characterised promoters of the pox virus group. For many of these promoters the time of the maximum transcription activity is already known.

A preferred promoter is the promoter of a vaccinia virus gene which codes for a 7.5 kD polypeptide (Cochran, M.A., Pucket, C. and Moss, B. (1985); "In vitro mutagenesis of the promoter region of a vaccinia virus gene; evidence for tandem early and late regulatory signal". J. Virol **54**, 30-37). The RNA synthesis for this 7.5 kD polypeptide takes place not only during the early transcription phase but also during the late transcription phase. The promoter designated p7.5 is thus an ideal promoter for a protein which is to be expressed uniformly over the entire cycle of the virus replication and expression.

A further very strong promoter, known from the vaccinia virus, is the promoter for a protein with a molecular weight of 11 kD, p11 (Bertholet, C., Drillien, R. and Witteck, R. (1985); "One hundred base pairs of 5′ flanking sequence of vaccinia virus late gene are sufficient to temporally regulate late transcription". Proc. Natl. Acad. Sci. USA **82**, 2096-2100). A particularly preferred embodiment, however, provides the use of avipox specific promoters. Promoters from the homologous system offer the greatest certainty of actual functionality.

An avipox promoter which is preferably used is a fowlpox promoter for a polypeptide with the molecular weight of 25 kD, namely the p25 promoter.

In addition to this preferred embodiment all other known fowlpox promoters can however also be used in accordance with the invention. All previously mentioned promoters can optionally be changed and improved by mutagenesis. Similarly, a synthetic promoter, which is recognised by the corresponding pox virus RNA polymerase, can be used for the insertion into the plasmid in accordance with the invention.

Most vaccinia virus late promoters, including the p11 promoter referred to above, have a characteristic functionally important sequence motif (A/TA/TTAAATGPuPu), which includes the translational initation codon ATG (Rosel, J.P. Earl, J. Weir, and B. Moss (1986) J. Virol. 60:436-449 and Magistris, L. and Stunnenberg, H.G. (1988) Nucl. Acids Res. 16, 3141-3156). Therefore for optimal expression foreign genes of interest one cloned just downstream of the ATG of the promoter. To facilitate the easy cloning of foreign open reading frames and to assure high levels of expression, a series of plasmid vectors can be constructed so that a gene fragment lacking its own ATG can be cloned in all three reading frames. Examples of cloning sites for use in such plasmids have already been described above.

According to the present invention a cloning site is used which enables insertion of foreign DNA immediately after an ATG initiation codon supplied by the promotor were after one or two purine (eg G) residues following the ATG codon. Preferably only one ATG codon is present. Such an arrangement will tend to be more efficient than, say, that described in WO-A-8802022, in which the translation initiation codon of the promotor is present as well as that of the inserted gene, because translational initiation usually occurs at the first ATG and although re-initiation at a second ATG does occur it is less efficient and the efficiency decreases as the distance between the two ATGs increases.

The DNA sequences flanking the promoter and the mcs or the foreign DNA inserted therein conveniently correspond to such portions of the avipox virus genome which are not essential for the function of this virus. The corresponding regions of the virus are destroyed by recombination with the plasmid in accordance with the invention and an open reading frame, which in the circumstances may be present, is thus cut. A careful selection of the sites provided for the insertion can for example result in the virulence of the virus strain used being reduced, although at the same time care must be taken to prevent the replication ability of the virus from being limited.

In the vaccinia virus system parts of the thymidine kinase gene have already been used successfully for flanking sequences. The recombinant virus in question is less virulent after loss of the thymidine kinase function than the parent virus (Buller, R.M., Smith, G.L., Cremer, K. Notkins. A.L. and Moss, B. (1985); "Decreased virulence of recombinant vaccinia virus expression vectors is associated with a thymidine kinase-negative phenotype"; Nature **317**, 813-815). In contrast to the orthopox system, the inactivation of the viral TK gene for selecting recombinant viruses, that is to say for selecting viruses with a TK⁻ phenotype, could not previously be used in the avipox system since there are no primary avian TK⁻ cells.

In a preferred embodiment a fowlpox virus is used as the avipox virus. The use of this virus has the advantage that its pathogenicity is strictly limited to avian cells. Many passages have, for instance, in the HP-1 strain led to an attenuation and a relatively strongly accelerated growth in chicken embryo fibroblasts (Mayr, A. and Malicki, K. (1966); "Attenuierung von virulentem Hühnerpockenvirus in Zellkulturen und Eigenschaften des attentuierten Virus"; Zentralblatt für Veterinarmedizin, column B, **13**; 1-13).
Plasmids which can be used in the preparation of recombinant avipox expression vectors useful in the invention can in addition to the components discussed above also carry a replicon (ori2) which permits the production of single stranded DNA. This additional function is useful for the checking of the construction of the plasmids and thus for example the insertion of foreign DNA in the mcs can be easily checked by inducing, in ways known in the art, the production of single stranded DNA which can subsequently be examined, using suitable primers, by the dideoxy sequencing method.

Examples of an ori2 which can be used in this manner are the replicons of filamentous phages, for instance of fd, f1 or M13. Of these three bacteriophages, M13 and its derivatives have become the best known since a whole series of sequencing vectors are constructed on it. The plasmids discussed above can be produced when using such an ori2 as a single strand and can be sequenced like a single strand phage.

The selection of a recombinant virus can be facilitated by the insertion of additional selection markers, for whose presence the transformed eukaryotic cells are tested. The use of a dominant selection marker (sm2) for the selection is particularly advantageous. The expression of this marker is preferably under the control of a strong, continuously active promoter, for instance p7.5. An example of such a dominant selection marker is the gpt gene which codes for the xanthine guanine phosphoribosyl transferase. This enzyme is a gene product from E. coli analogous to hypoxanthine guanine phosphoribosyl transferase from mammalian cells. Like the mammalian cell enzyme it catalyses the condensation of phosphoribosyl pyrophosphate with hypoxanthine or guanine into inosinic or guanic acid (IMP or GMP). Furthermore, the bacterial enzyme also has the property of being able to convert xanthine into XMP, a reaction which the enzyme from mammalian cells cannot perform or can only perform poorly (Mulligan, R. and Berg, P. (1981); "Selection for animal cells, that express the E. coli gene coding for xanthine-guanine phosphoribosyl transferase"; Proc. natl. Acad. Sci. USA **78**, 2072-2076). A recombinant virus, which carries the gpt gene, can thus, in the presence of mycophenolic acid, render competent mammalian cells infected with this virus by providing XMP for the synthesis of GMP and thus dGTP and DNA (Falkner, F.G. and Moss, B. (1988), "Escherichia coli gpt gene provides dominant selection for vaccinia virus open-reading-frame expression vectors", J. Virol. **62**, 1849-1854 (1988)), whilst in non-infected cells synthesis of XMP is inhibited by the mycophenolic acid; the use of xanthine as a substrate is however not possible by reason of the different specificity of the analogous mammalian cell enzyme. The wild-type virus does not grow in the presence of mycophenolic acid.

An alternative for the expression of the gpt gene as selection marker is the use of the gene for the hygromycin B phosphotransferase. Hygromycin B is an antibiotic synthesised by streptomycetes which inhibits protein synthesis both in prokaryotes and also in eukaryotes by blocking ribosomal translocation and interfering in the aminoacyl-t-RNA recognition. This antibiotic can be inactivated by a phosphotransferase isolated from E. coli (Blochlinger, K. and Diggelmann, H. (1984); Mol. and Cell. Biol. **4**, p 2929-2931).

A further alternative for selection procedures for the identification of recombinant virus is the co-expression of the E. coli lacZ gene and screening for in vivo expression of the enzyme beta-galactosidase ('blue plaque screening'). Examples of vaccinia virus insertion vectors that allow blue plaque screening are vectors pSC11 (Chakrabati, S., Brechling, K. and Moss, B. "Vaccinia virus expression vector: coexpression of beta-galactosidase provides visual screening of recombinant virus plaques", Mol. Cell. Biol. **5**, 3403-3409 (1985)) and pUV 1 (Falkner, F.G., Chakrabati, S. and Moss, B. "pUV 1: a new vaccinia virus insertion and expression vector" Nucl. Acids Res. **15**, 7192 (1987)).

DNA coding for the desired proteinaceous material can be inserted into the mcs of the plasmids described above. Complete genes, ie genes whose translation initiation codons and translation termination codons are also transferred can, for instance, be inserted. A peculiarity of all pox virus systems is, however, that pre-splicing of MRNA does not appear to take place. This is presumably due to the fact that all virus-dependent processes take place in the cytoplasm of the infected cell whilst the cellular splicing apparatus is in the cell nucleus. Care should therefore be taken when reconstructing the plasmids, which are to be used for recombination with avipox virus, that intron-free DNA is used when using eukaryotic genes.

For many purposes it will be appropriate not to insert the complete gene for a protein into the recombinant virus via a plasmid but instead, for instance, only to insert the coding sequences for functional regions and/or antigenic determinants of the protein. In certain cases the replicability of the virus could be reduced, eg by the insertion of a complete gene, or the cell prematurely excessively damaged. Additionally, only portions of the protein are in many cases necessary for certain purposes.

DNA inserted into the plasmid can be not only of natural origin but also synthetic. DNA of natural origin will be either genomic DNA isolated, for instance, from the genome of prokaryotes or cDNA of complex, eukaryotic genes. Synthetic DNA is as a general rule prepared from synthetically produced oligonucleotides and subsequently introduced into the plasmid. Synthetic DNA has the advantages that it can be modified during production in accordance with subsequent requirements and that the restriction sites used for the cloning can be built in terminally.

Proteinaceous materials produceable by the invention include proteins and compounds with a protein or polypeptide content such as glycoproteins, acetylated proteins, carboxylated proteins and other post-translationally modified proteins. The vitamin-K dependent blood factors are examples of post-translationally modified proteins.

A preferred application of the invention is the production of proteinaceous materials with the biological function of at least one of the following proteins: one of the blood clotting factors VIII, IX, XII, XIII or S, von Willebrand factor, protein C, prothrombin, antithrombin III, plasminogen, hirudin, NGF, FGF, TNF, B-cell stimulating factors, proteins from the interleukin group, apolipoproteins or glycoproteins from viruses. A substantial advantage of the production of these proteins from the avipox expression system is the fact that, for example, the blood clotting factors (which are conventionally recovered from blood sera) are not contaminated with human-pathogenic viruses such as HIV. Furthermore, the recovery of these factors from cell culture makes significant enrichment possible.

A particular subgroup of post-translationally modified proteins which may usefully be produced by the invention are the vitamin-K dependent blood factors, as mentioned above, which include Factors VII, IX and X, proteins C and S and prothrombin. Prothrombin has been extensively studied in terms of gene organisation and its biological function. Correct post-translational modification is a prerequisite for activity, and it appears that commercially available cells such as monkey kidney, human liver, etc may allow synthesis of fully or at least adequately carboxylated prothrombin in the pox virus expression system. This type of modification is the most important for all blood factors. In addition, secretory cells, if they become available and allow infection with an avipox virus such as FPV, could become promising cells for use in the invention.

It should be noted that production of recombinant avipox virus useful in the invention and produced using plasmids as described above occurs in vivo by recombination of the flanking avipox sequences of the plasmid with the avipox virus. The originally intact viral gene is destroyed by the recombination and thus inactivated.

Other preferred proteinaceous materials for production by means of the invention are proteins or their derivatives from pathogens; these can be used for example for immunisation purposes. Examples include pertussis, tetanus, malaria, AIDS, tick-borne encephalitis, hepatitis B and herpes infections. In most cases the proteins to be used for the immunisation are proteins of the pathogens in question inducing neutralising antibodies or inducing cytotoxic T-cells.

Recombinant avipox viruses useful in the invention include a foreign gene (or parts thereof or other DNA coding for the desired proteinaceous material) under the control of a promoter in addition to the natural avipox components. The promoter may be an additional promoter, Which does not naturally occur in avipox viruses. Depending on the nature of the promoter, the foreign gene is transcribed either during the early transcription phase of the virus, during the late transcription phase or during the entire transcription period.

The recombination is preferably performed with fowlpox virus, which is an avipox virus, as repeatedly passaged avipox (for example the HP-1 strain) yields a highly attenuated strain which is adapted to the requirements of cell culture.

The resulting transcripts are translated in the cytosol of the infected cell and subsequently post-translationally modified, eg glycosylated or acetylated. The system in accordance with the invention can thus be used to express eukaryotic DNA sequences which are foreign to pox viruses and results in authentically modified proteins.

Proteinaceous materials which may preferably be produced with this system eg, as already mentioned above, one of the blood clotting factors VIII, IX, XII, XIII or S, von Willebrand factor, protein C, prothrombin, antithrombin III, plasminogen, hirudin, NGF, FGF, TNF, B-cell stimulator factors, proteins from the interleukin group, apolipoproteins or viral glycoproteins, for instance of the TBE virus.

Recombinant avipox viruses useful in the invention can, depending on their application, be maintained either in vivo in birds or in vitro in cell cultures. For long term storage it is recommended that cell cultures infected with the viruses in accordance with the invention be frozen or lyophilised.

Expression in a process of the invention will, at the present state of technology, usually take place in tissue culture, although the use of transgenic animals is not excluded. Animal tissue cultures which may be suitable for use in the invention include both mammalian and avian cell cultures.
Various mammalian cell types of different origin or stage of differentiation may be used for expression of blood factors if they allow uptake of an avipox virus and an abortive infectious cycle. The need for optimal conditions within the cells in regard to exact folding of the protein, exact glycosylation, acetylation or vitamin K dependent carboxylation and even secretion of the synthesised protein is of course a major consideration for successful expression studies.

Even if mammalian cells do not allow avipox virus to replicate completely, this semi-permissiveness of cells and the possibility that cells are not damaged by the virus might well be an advantage. Infected cells which are not dying, ie that do not suffer from a host-shut off, might efficiently synthesise the foreign gene of interest. Because of the semi-permissiveness of mammalian cells, early avipox (eg FPV) promoters may be particularly suitable for the regulation of expression. Early promoters are active, even if late stages of replication and therefore "late" RNA synthesis cannot take place.

Mammalian cells are particularly useful for expression since a great variety of these cells is commercially available. In contrast, only one or two permanently growing avian cell lines, established from quail or duck, can be obtained from depository institutions such as ATCC or GIBCO. "Immortalised" avian cells which are well suited for expression studies with avipox viruses may become available.

Mammalian cell cultures which may be used can in principle either be first generation (ie "primary") cells or immortalised cells. First-generation mammalian cells, however, are not in practice likely to become commercial producer cells for avipox expression systems as they have a very limited lifespan in culture. Permanently growing cells are at present necessary in order to test cells for optimal expression and secretion of proteinaceous componds such as, for example, blood factor proteins. Suitable stable, transformed cells include BSC-1, CV-1, MRC-5, Vero and human HepG2 cells.

A further advantage of immortalised cells is that they can be propagated in readily available serum-free medium whereas primary cells are much more dependent on growth factors supplied by foetal calf serum, although synthetic media might be used for primary cells in the future. At present, synthetic media for mammalian primary cells are very expensive and the efficiency of growth is questioned by some workers.

Avian cells are naturally those cells for optimal propagation of avipox virus such as FPV in contrast to permanently growing mammalian cells. In addition, chick embryo fibroblast (CEF) cells are primary cells and licensing authorities allow these cells for production of vaccines (for example, TBE), even though endogenous retroviruses are present in the genome of chicken cells. Immortalisation or "growth-transformation" of mammalian cells is either the result of genetic damage to the cellular genome or the cells are "transformed" by a DNA virus (such as SV-40 or Epstein Barr Virus (EBV)).

Production of proteinaceous materials such as blood factors in primary avian cells such as CEF cells infected with avipox virus as an expression vector is a useful means for production since the shut-off of host cell protein synthesis proceeds much more slowly (5 to 7 days) than in cells infected with vaccinia virus (2 to 3 days).

Additional avian cells such as quail cells, duck embryonic cells or "haematopoetic" cells may also be suitable as expression cells, particularly if growth in suspension culture were achieved as this would facilitate large scale production of any cloned blood factor or other proteinaceous material.

A cell culture which is preferably used comprises primary chicken embryo fibroblast cells.

In cell cultures useful in the invention, the peptide or polypeptide component of the proteinaceous molecule coded by the foreign DNA may be synthesised continuously or only at certain times in the virus cycle in dependence on the promoter used. Proteinaceous materials thus produced can be harvested from the cells by any suitable technique, for example those known in the art, and purified using the usual methods of protein chemistry. The proteinaceous material will therefore generally be free of avipox virus material and usually generally free of cell components.

A proteinaceous material produced in accordance with the invention can be used as a diagnostic agent. In this case it would be possible to detect antibodies against certain proteins of a pathogenic virus in infected individuals by an antigenic-antibody reaction between the peptide or polypeptide produced in accordance with the invention and the serum of the human or animal (eg mammalian) patient.

For a bettter understanding of the invention, and to show how it may be put into effect, reference will now be made, by way of example, to the accompanying drawings, in which:
Figure 1 illustrates the construction of the fowlpox virus insertion vectors pFPtk-gptFI and pFPtk-gptFII;

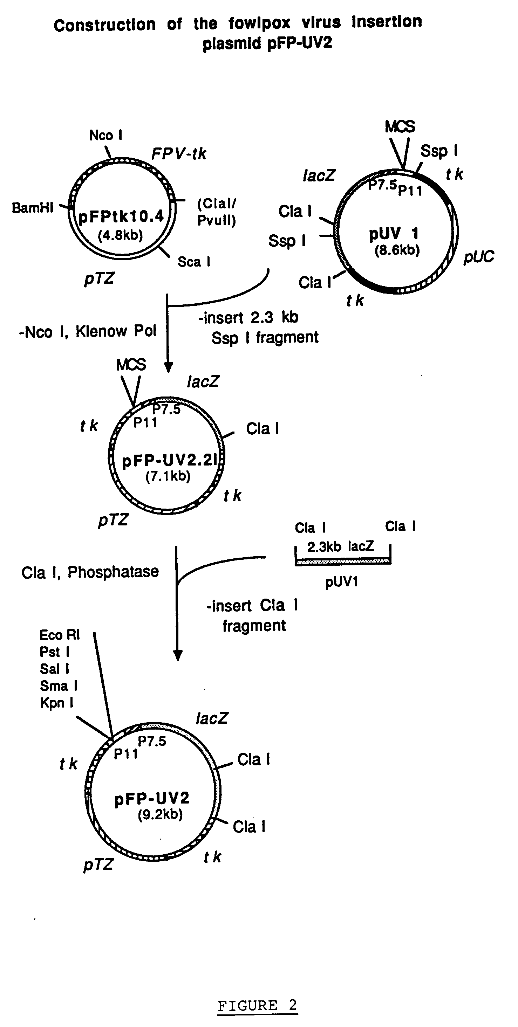

Figure 2 illustrates the construction of the fowlpox virus insertion vector pFP-UV2;
Figure 3 illustrates the construction of the recombinant fowlpox virus vfp-lacZ; and
Figure 4 illustrates the construction of the recombinant fowlpox virus vfp-PTHB.

The following examples further illustrate the invention:

### Example 1

### Production of Primary Bird Cells from Embryonic Specifically Pathogen-Free Eggs (SPF).

After an incubation time of 12 days SPF chicken eggs (approximately 2000 eggs daily) were tested with an egg lamp, disinfected with alcohol and opened in a sterile box. The living embryos were removed with tweezers, collected in a steel vessel, freed of remaining portions of egg by washing with a TBE trypsinating buffer and so distributed in 2 l beakers that about 500 embryos were apportioned to each beaker. The embryos were then pressed through a metal sieve in a pneumatic press.

### Extraction of the chicken embryo cells

About 500 sieved embryos were placed in a glass vessel of the trypsinating device which contained about 7 l of the TBE trypsinating buffer. The major proportion of the erythocytes and blood components were washed out with a further 10 l of the same buffer. For this purpose the trypsinating devices were equipped with a stirrer, pumps and a thermostatically controllable water bath. The sample was stirred well and 200 ml trypsin solution (45g trypsin was added to 1 N HCl to 1 l to produce this solution), 40 ml DNAse solution (450 U/ml), 14 ml neomycin sulphate solution (25 g/l) and 7 ml polymyxin solution (2.5 x 10⁵ U/ml) were added. The pH was adjusted to 7.4 with filtered NaOH and checked with a suitable test paper.

The trypsinating procedure lasted about 35 min at 37^{o}C. The progress fo the trypsination was tested at regular intervals in glass tubes. After termination of the trypsination the content of the trypsinating device was pumped into a 20 l decanter and diluted with about 12 l TBE trypsinating buffer (0.125% trypsin in TBE). The cell suspension was filtered through a metal filter at the outlet of the trypsinating device and a gauze filter at the inlet of the decanting vessel. The contents of the decanting vessel were mixed well with a magnetic stirrer and a Teflon-coated stirring flea. The trypsinating device was flushed with 10 l of the TBE trypsinating buffer which was subsequently centrifuged on route to the decanting vessel. A proportion of the cell suspension was fed directly from the decanting vessel into a continuous flow centrifuge and centrifuged at 8500 rpm (17100 x g). 400 ml of the cell suspension was pumped into the centrifuge per minute. Approximately 75 minutes were required altogether for 30 l. A second portion of the cell suspension was introduced into 1 l plasic centrifuge beakers and centrifuged at 1000 rpm (1650 x g) for 25 minutes. This division into two centrifuges was necessary due to the fact that the capacity of the continuous flow centrifuges was too small. About 250 ml of the cell suspension was removed from the decanting vessel and the supernatant and the cells was tested on foreign viruses.

### Purification of the chicken embryo cells

200 ml medium per preparation was prepared in a steel vessel or in 20 10 l bottles. 10 ml of it, or in the case of the bottles 20 x 10 ml, was removed for sterility tests. 100 ml was kept at 4^{o}C as samples and 25 ml was used for the pyrogenicity test. An identity test was also carried out by determining the gentamycin activity.

The cells obtained by the centrifugation with the continuous flow or fixed angle rotor were resuspended in 2 l TBE medium and stirred in a glass Erlenmeyer flask with a magnetic stirrer at room temperature for 30 minutes. After the centrifugation (25 minutes at 1000 rpm, 20^{o}C) the supernatant was decanted, the precipitate resuspended in 2 l TBE medium and again centrifuged under the same conditions. The supernatant was again decanted, the cells resuspended in 1 l TBE medium and stirred for 45 minutes at room temperature.

The cells were filtered through sterile gauze before dissemination and then disseminated thinly so that they were confluent after 2 days incubation at 37^{o}C.

### Example 2

### Isolation of Fowlpox Virus (FPV) DNA from a Non-Essential Region of the Fowlpox Virus Genome.

The TK gene was selected as the non-essential region of the genomic fowlpox DNA. The isolation of the FPV-TK gene includes the steps of the infection of primary chicken embryo fibroblasts (CEF) and purification of FPV by centrifuging and also the isolation of viral DNA and removal of viral restriction fragments onto agarose gels and the identification of the TK gene by hybridisation with an oligonucleotide specifically radioactively marked for the TK gene.

### a) Infection of primary CEFs

Primary CEFs were produced, as described in a medium containing DMEM supplemented with glutamine, 2% foetal calf serum and antibiotics. Confluent individual cell layers of the CEFs (1 x 10⁸ cells) were infected with 5 plaque forming units (pfu) per cell of an unpurified strain solution of the strain HP-1. After 4-5 days incubation at 37^{o}C in a medium (see Example 1) the cells were scraped away, centrifuged (10 minutes, 1000 x g) and the pellet resuspended in 10 mM Tris HCl pH 9.0. The cells were homogenised (for instance in a Dounce homogeniser with 20-30 pestel movements), the nuclei were centrifuged away by 5 min centrifugation at 800 x g and the virus-containing supernatant subjected to ultrasound in order to disperse the virus. In order further to purify the virus the supernatant was centrifuged on a gradient of 36% saccharose, 10 mM Tris HCl pH 9.0 (Beckman SW27 tubes; 14000 rpm, 80 min at 4^{o}C). The virus pellet was resuspended in 1-2 ml 1 ml Tris HCl pH 9.0, subjected to ultrasound and further purified on a continuous saccharose gradient (24-40% saccharose, 1 mM Tris HCl pH 9.0). 1 ml of the virus suspension was applied on a gradient prepared in a Beckman SW28 centrifuge tube and centrifuged for 50 min with 12000 rpm at 4^{o}C. The virus band was pipetted off, diluted with 2 vol 1 mM Tris HCl pH 9.0 and centrifuged at 13500 rpm for 60 min at 4^{o} in the SW28 tube. The pellet was resuspended in 1 mM Tris HCl pH 9.0 and frozen at -70^{o}C.

Virus purified in this manner is referred to below as "purified virus".

### b) Isolation of FPV-DNA and characterisation of viral restriction fragments on agarose gels.

Purified FPV was resuspended in a solution of 10 mM Tris HCl, 10 mM Na₂-EDTA, 10 mM NaCl, 0.5% SDS, pH 8.0, and incubated overnight in the presence of 100 µg/ml proteinase K. The FPV-DNA solution was carefully extracted once with phenol and twice with chloroform and precipitated with ethanol. The pellet was washed once with 70% ethanol and resuspended in a solution of 10 mM Tris, 1 mM Na₂-EDTA.

DNA isolated in this manner from purified FPV is referred to below as "purified FPV-DNA".

Aliquots of 2 µg purified FPV-DNA were cleaved with restriction endonucleases and separated onto 0.6% agarose gels. The fragment pattern of the DNA of the FPV HP1 strain is published (Müller, H.K., Wittek, R., Schaffner, W. Schümperli, D., Menna A. and Wyler, R. (1978), Comparison of five pox virus genomes by analysis with restriction endonucleases Hind III, BamHI and EcoRI, J. Gen. Virol. **38**, 135-147).

### c) Identification of the TK gene by hybridising with a radioactively marked oligonucleotide specific for the TK gene.

The restriction fragments separated in Example b) were transferred to nitrocellulose filters (Southern blot) and hybridised with a 28mer oligonucleotide (5'-CAG TTA TTG TGG CCG CGC TTA ACG GTG A-3'); the 28mer sequence originates from a conserved portion of the fowlpox TK gene sequence (Boyle, D.B., Coupar, B.E.H., Gibbs, A.J., Seigman, L.J. and Both, G.W. (1987), Fowlpox virus thymidine kinase: nucleotide sequence and relationship to other thymidine kinases, Virology **156**, 355-356).

The 28mer was previously radioactively marked with gamma-³²P-ATP (Amersham 2000 Ci/mmol; 10 mCi/mcl); for this purpose 20 picomol oligonucleotide in 20 µl reaction volumes were marked with polynucleotide kinase in accordance with the instructions of the manufacturer (Boehringer Mannheim).

The hybridisation was effected overnight in 6 x SSC, 10 x Denhardt solution 0.2% SDS at 68^{o}C.

The filter was washed four times for 30 min in 3 x SSC, 0.1% SDS at 50^{o}C and subsequently autoradiographed.

The TK gene could be unambiguously identified in this manner and associated with the respective restriction fragments. The appropriate restriction fragment was used in Example 3 as follows.

### Example 3

### Construction of fowlpox virus insertion plasmids pFPtkgpt-FI and pFPtkgpt-FII

To construct the insertion plasmids pFPtkgpt-FI and pFPtkgpt-FII, fowlpox virus (strain HP1 München) DNA was isolated as described in Example 2 above.

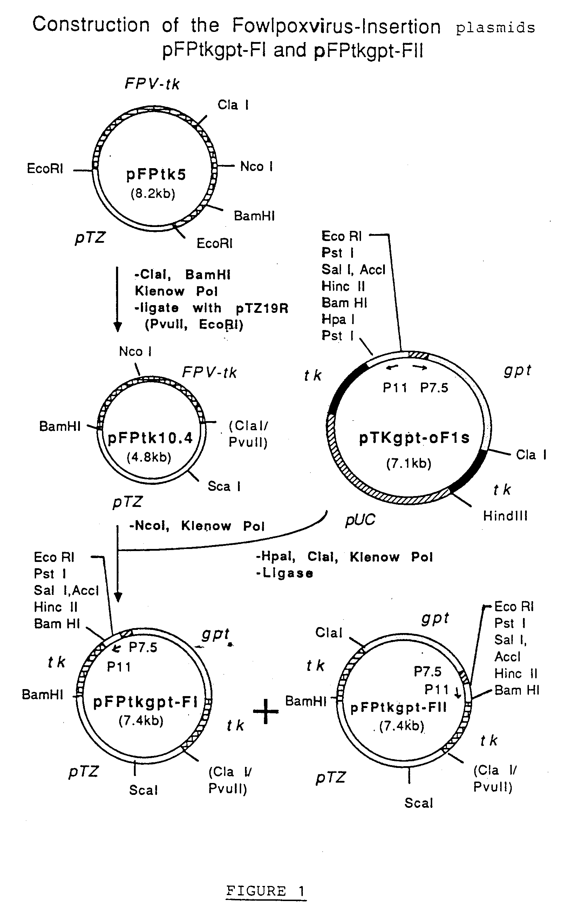

As shown in Figure 1, an Eco RI digest of fowlpox virus DNA was cloned into the EcoRI site of the vector pTZ19R. The plasmid pFPtk5 was identified by colony filter hybridisation using the oligonucleotide probe 5'-CAG TTA TTG TGG CCG CGC TTA ACG GTG A-3' (Example 2(c)). The plasmid contained a 5.5 kb EcoRI fragment; it was cleaved with Cla I, BamHI and Sca I, treated with Klenow polymerase and ligated with the vector pTZ19R, which had been treated with PvuII, EcoRI and phosphatase. The resulting plasmid, pFPtk10.4, had the 2.3 kb BamHI-ClaI insert, which contains the fowlpox virus tk-gene (Boyle, D.B. and Coupar, E.H. (1986). "Identification and cloning of the fowlpox virus thymidine kinase gene using vaccinia virus". J. Gen. Virol. **67**, 1591-1600 and Boyle, D.B., Coupar, B.E.H., Gibbs, A.J., Seigman, L.J. and Both, G.W. (1987). "Fowlpox virus thymidine kinase: nucleotide sequence and relationships to other thymidine kinases", Virology **156**, 355-356). The Hpa I, ClaI and Klenow polymerase treated gene cassette (containing the Eco-gpt gene under control of the vaccinia virus P7.5 promoter (Cochran, M.A., Puckett, C. and Moss, B. (1985) J. virol. **54**: 30-37) and the vaccinia virus P11 promoter (Bertholet, C., Drillien, R. and wittek, R. 1985 Proc. Natl. Acad. Sci. USA **82**:2096-2100.) with adjacent multiple cloning sites) of the plasmid pTKgpt-oF1s (Falkner, F.G. and Moss, B. (1988) J. Virol. **62**, 1849-1854.), was inserted into the unique, Klenow polymerase-treated Nco I site of plasmid pFPtk10.4 resulting in the plasmids pFPtkgpt-FI and pFPtkgpt-FII.

### Example 4

To construct pFP-UV2, the 2.2 kb SspI fragment of the vaccinia virus insertion plasmid pUV 1 (Falkner, F.G., Chakrabati, S. and Moss, B. "pUV 1: a new vaccinia virus insertion and expression vector" Nucl. Acids Res. **15**, 7192 (1987)) was used. This fragment includes the vaccinia virus promotors p11 and p7.5 and part of the E. coli lacZ gene. The fragment was prepared out of low melting agarose and cloned into the unique Klenow polymerase-treated NcoI site of the plasmid pFPtk10.4 (see Figure 1) resulting in the plasmid pFP-UV2.2i. The 2.3 ClaI fragment (that contains part of the lacZ gene) prepared from the plasmid pUV 1 was inserted into the unique ClaI site of pFP-UV2.2i resulting in plasmid pFP-UV2. This plasmid allows the expression of open reading frames downstream of the strong vaccinia virus p11 promoter and contains the E. coli lacZ marker gene driven by the vaccinia virus p7.5 promoter. The fowlpox tk flanking regions direct the expression cassette into the viral tk-locus.

### Example 5

### Production of insertion vectors which contain a pox virus promoter, individual restriction sites for the insertion of foreign DNA and flanking DNA sequence from an avipox virus genome.

The vector used for the construction of the insertion vector can be any common plasmid, for instance pBR322 and its derivatives and pUC7 and its derivatives, any cosmid or any phage.

Two types of insertion vectors were constructed;
a) vectors with pox promoters (Type 1 promoter)
b) vectors with pox promoters followed by a translation-iniation codon, restriction endonuclease cleavage points and stop codons (Type 2 promoter).

Each promoter fragment was ligated directly or after appropriate modification of its ends in a plasmid linearised with a suitable restriction enzyme. The ligation of the sticky or blunt ends was effected in accordance with the standard method. The promoter was preferably introduced into a plasmid, eg pUC19 which already contained a multi-faceted mcs in the "downstream" region of the promoter. The Type 2 promoters were completed by the additional insertion of synthetic oligodeoxyribonucleotides, which contained one or more stop codon. The promoter-containing plasmids produced in this manner were amplified after transformation into bacteria, subsequently isolated and purified. The relevant promoter fragment with adjacent restriction endonuclease cleavage sites and optionally translation signals were excised from these plasmids by cleavage with restriction endonucleases and purified in accordance with the standard method.

The DNA flanking the promoters and the newly introduced restriction endonuclease cleavage sites from a non-essential region of the avipox virus genome, in this case the DNA coding for the thymidine kinase gene, was identified and subcloned, as described in Example 2c. Any selectable plasmid capable of replication in prokaryotes or eukaryotes can be used for this subcloning. A preferred plasmid is pTZ19R because this has an additional replicon for single strand packing. The cloned fragment was subsequently ligated with a suitable restriction endonuclease into the resulting cleavage site and the promoter cassette, comprising the Type 1 or Type 2 promoter defined above, optionally in conjunction with mcs, was ligated into the resulting cleavage site.

In this manner two different types of plasmids were obtained, the Type 1 and Type 2 plasmids.

### Example 6

### Insertion of a foreign gene into a Type 2 insertion plasmid.

In order to be able to assay the expression of a foreign gene in the avipox system qualitatively and quantitatively, the E. coli lacZ gene, which codes for the enzyme beta-galactosidase, was cloned into the vector pFPtk-gptFl. For this purpose 5 mcg pFPtk-gptFl plasmid DNA was linearised with the restriction endonuclease BamHI and treated with alkaline phosphatase (Boehringer) in accordance with the instructions of the manufacturer. The lacZ gene is derived from the plasmid pMC 1871 (Shapira, S.K., J. Chou, F.V. Richaud and M.J. Casadaban (1983); "New versatile plasmid vectors for expression of hybrid proteins coded by a cloned gene fused to lac Z gene sequences encoding an enzymatically active carboxyterminal portion of beta-galactosidase"; Gene **25**, 71-82) and was isolated as the BamHI fragment and cloned into pFPtk-gptF1 in accordance with standard procedure. The lacZ BamHI fragment has no ATG translation initiation codon of its own and requires the ATG codon supplied by the vaccinia virus P11 promoter for expression. Ligation of the BamHI fragment led to an insertion in the correct reading frame.

The construction and isolation of a recombinant avipox virus, which is beta-galactosidase positive, was carried out as described in Examples 7 and 8.

### Example 7

### Transfection of avian cells with plasmids which contain chimeric genes.

Plasmids in accordance with the invention, which contain the chimeric genes flanked by non-essential DNA from the avipox virus genome, were used for the transfection of cells which had already previously been infected with wild type avipox virus. The chimeric gene was subsequently inserted into the avipox virus genome by homologous recombination. Confluent cell monolayers of primary chicken embryo fibroblasts (CEF) or other avian cells were generally infected in culture flasks with a bottom surface of 25cm² with 0.01 to 0.50 plaque forming units (pfu) avipox virus per cell. About 5 µg of plasmid DNA, either alone or together with 1 µg avipox virus DNA were mixed in each case with 14 µg calf thymus DNA or another carrier DNA in 1 ml 0.1% dextrose, 0.14 M NaCl, 5 mM KCl, 2 mM Na₂HPO₄, 20 mM Hepes, pH 7.05 and precipitated by the addition of CaCl₂ down to a final concentration of 125 mM. The mixture was carefully mixed and incubated for about 15 min at room temperature. Five hours after the infection 1 ml of the finally distributed suspension was added to an infected cell monolayer from which the medium had previously been removed. After 30 minutes 10 ml medium or 10 ml of another tissue culture medium with a content of 8% foetal calf serum was added to each flask and the incubation continued at 37^{o}C. Four hours after the infection fresh medium with a content of 8% foetal calf serum was supplied and the incubation continued for 96-120 hours. After this time, the infected cells were scraped out of the flask, centrifuged, resuspended in tissue culture medium and digested with the same volume of 2.5% trypsin solution in order to liberate the virus.

### Example 8

### Selection and isolation of recombinant avipox viruses and detection of a foreign gene product.

The viruses isolated from transfected cells comprised a population which contained only a small percentage of recombinants. Selective and non-selective methods were used for the isolation of these recombinants.

The selective method is based on the ability of the recombinants to grow under those conditions under which the growth of the original virus is inhibited. This was achieved by using an insertion plasmid which in addition to the first chimeric gene included a second chimeric gene, which comprised a vaccinia promoter in conjunction with the bacterial gpt gene and which was also flanked by the non-essential avipox sequences. After homologous recombination in vivo both chimeric genes are present in a non-essential region of the avipox virus DNA. The viruses subsequently express the bacterial gpt gene which functions as the dominant selection marker. The conditions for the selective isolation of avipox recombinants are described below: firstly, the gpt gene containing virus population was amplified by passaging the virus under selective conditions at low multiplicity. CEF monolayers were infected with different dilutions of the trypsinated virus-containing cell suspension (at multiplicities of infection of 0.1 and 0.01) as described in Example 7, and grown for six days under selective conditions (selection medium: DMEM, 2,5% foetal calf serum, 750 ng/lm mycophenolic acid, 250 µg/ml xanthine, 15 µg/ml hypoxanthine). The amplification procedure was repeated three times. The final identification of the recombinants was done by a plaque assay as follows: different cultures of a viral crude stock consisting of the trypsinised cell suspension were used for the infection of CEF monolayers. Thereafter the cells were coated with a selection medium (comprising DMEM, 2.5% foetal calf serum, 750 ng/ml mycophenolic acid, 250 µg/ml xanthine, 15 µg hypoxanthine and 1% low melting point agarose). After 4 to 5 days at 37^{o}C in a moist atmosphere with 5% CO₂, the plaques were detected by colouring with 0.005% neutral red. Several plaques were picked and subsequently identified by dot blot hybridisation.

DNA-DNA hybridisations were, for instance, used as non-selected methods for the identification of virus plaques which contain recombinant viruses. The detection was preferably carried out by dot blot hybridisation. In this method, the virus obtained after transfection of already infected cells with chimeric plasmids was seeded out onto a cell monolayer with 1% agar coating. After 96 hours the plaques were detected by colouring with neutral red. Viruses from individual plaques were removed using a sterile Pasteur pipette and used to infect CEF cell monolayers in depressions with 16mm diameter in microtiter plates. After 48 hours incubation at 37^{o}C the cells were scraped off, lysed by three cycles of freezing and thawing, and collected on nitrocellulose filters by filtration with a micro sampling device (Schleicher and Schüll). The filters were then washed with 100 mM NaCl, 50 mM Tris-HCl, pH 7.5 and laid out three times successively on Whatman 3MM paper which had been soaked on the first occasion with 0.5 M NaOH, on the second occasion with 1M Tris-HCl, pH.7.5 and on the third occasion with 2 x SSC (1 x SSC = 0.15 M NaCl, 0.015 M sodium citrate), subsequently baked for 2 hours at 80^{o}C and then, supplemented with 0.1 mg/ml denatured salmon sperm DNA, incubated for 4 hours with 5 x Denhardt solution (Biochem. Biophys. Res. Commun. 23, 631-646, 1966) in 4 x SSC at 65^{o}C. The following DNA, which had been marked by "nick translation" with ³²P, and sodium dodecyl sulphate (SDS) were added in a final concentration of 0.1% and the hybridisation continued for 12 hours. The filter was then washed twice for 15 min at 65^{o}C with 2 x SSC/0.1% SDS and then with 0.2 x SSC/0.1% SDS. The filters were subsequently autoradiographed and a recombinant virus identified by reference to film darkening.

An alternative method has been described by Villarreal and Berg (Science 196, 183-185, 1977). For this method a replica of the virus plaque is obtained by laying a nitrocellulose filter on the cell monolayer. A DNA-DNA hybridisation is carried out with this cellulose filter, as described above. After localisation of the plaques, which contain a recombinant virus, the remaining viruses are isolated from the agar which was originally layered on the plaques.

In the case of beta-galactosidase-expressing recombinants, blue plaque screening was performed instead of gpt selection as follows: CEF monolayers grown in large petri dishes (15 cm diameter) were infected at low multiplicities (usually the 10⁻³, 10⁻⁴ and 10⁻⁵ dilutions of the non-amplified viral crude stock) and grown for two days in medium (MEM, containing 2% foetal calf serum, glutamine, penicillin and streptomycin) and then an overlay of the same medium containing 1% low melting point agarose was applied for another three days. Recombinant (blue) plaques were detected by staining with an overlay consisting of 0.4 mg/ml X-Gal, 0.005% neutral red and 1% agarose in phosphate buffered saline.

### Example 9

### Construction of the recombinant fowlpox virus vfp-lacZ

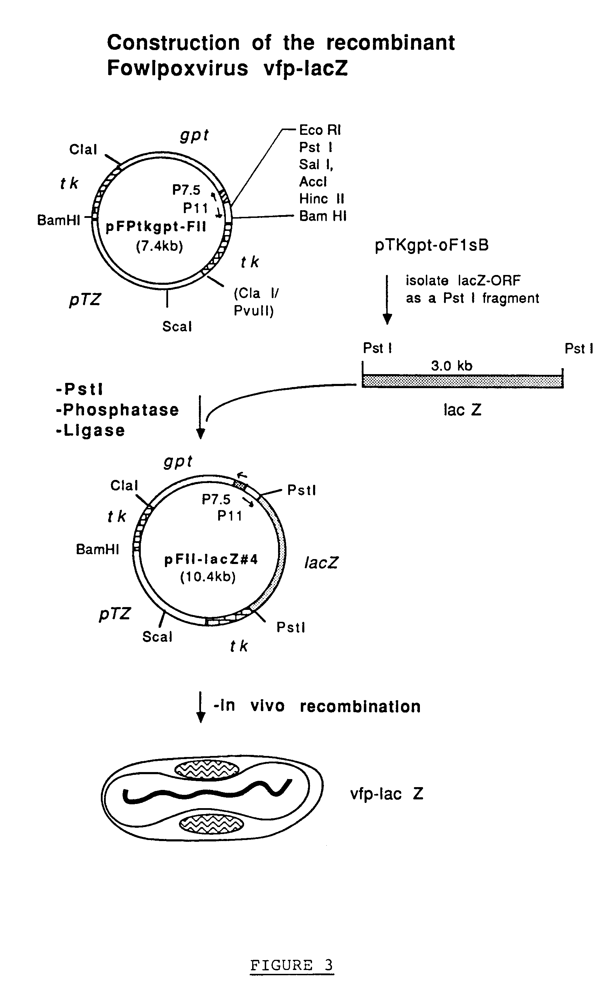

As shown in Figure 3, to construct the viral recombinant vfp-lacZ, the 3.0 kb Pst I fragment containing the E.coli lac Z gene, was purified out of low melting agarose from a Pst I digest of the plasmid pTKgpt-of1beta (Falkner, F.G. and Moss, B. (1988) J. Virol. **62**, 1849-1854) and inserted into the unique Pst I site of the insertion plasmid pFPtkgpt-FII. The two resulting orientational isomers were designated pFII-lacZ#4 and pFII-lacZ#7.

To prove that, in pFII-lacZ#4, the lacZ gene was correctly orientated and inserted in frame behind the initiation codon of the vaccinia virus P11 promoter, a transient expression assay (as described by Cochran, M., Mackett, M. and Moss, B. (1985) Proc. Natl. Acad. Sci. USA **82**, 19-23) was performed. Only extracts of cells transfected with pFII-lacZ#7 contained high beta-galacosidase activities, while extracts obtained with pFII-lacZ were non-active.

To construct the recombinant fowlpox virus, confluent monolayers of primary chicken embryo fibroblasts (CEF) were infected with 0.02 pfu per cell of the fowlpox virus strain HP1 (provided by A. Mayr, Munich). After an adsorption period of one hour, medium was added and the incubation was continued for another 3 hours. A DNA calcium phosphate precipitate consisting of 5 mcg of pFII-lacZ#4, 14 µg of herring sperm carrier DNA and 1 µg of fowlpox wildtype virus DNA was laid over the cells. After 15 min of incubation at room temperature, 9 ml of medium (DMEM, 8% FBS with antibiotics) were added. The medium was changed after 4 h and the incubation was continued for another 3 days. Cells were harvested, and a viral crude stock was prepared by resuspending the cells in 500 µl of medium and adding an equal volume of 2.5% of trypsin. After 30 min of incubation, 500 µl of foetal calf serum was added to neutralise the trypsin.
For the plaque assay chicken embryo fibroblasts were seeded at a density of 1 x 10⁵ cells per well of a 6-well plate (well diameter 3 cm) and grown to confluency (usually for 2 days). The wells were infected with 500 µl of the 10⁻³, 10⁻⁴ and the 10⁻⁵ dilutions of the viral crude stock, amplified (as described in Example 8) and incubated for adsorption for 1 h. After two days growth in gpt-selective medium (DMEM, 2.5% FBS, antibiotics, 750 ng/ml MPA, 250 µg/ml xanthine and 15 µg/ml hypoxanthine), the cells were overlaid with gpt-selective medium containing 1% low melting agarose and grown for another two days. The cells were then stained with an overlap containing 0.005% neutral red and 0.4 mg/ml X-Gal. After 5 h of incubation blue plaques were readily visible, indicating beta-galactosidase expressing viral recombinants.

### Example 10

### Construction of the fowlpox virus vfp-PTHB

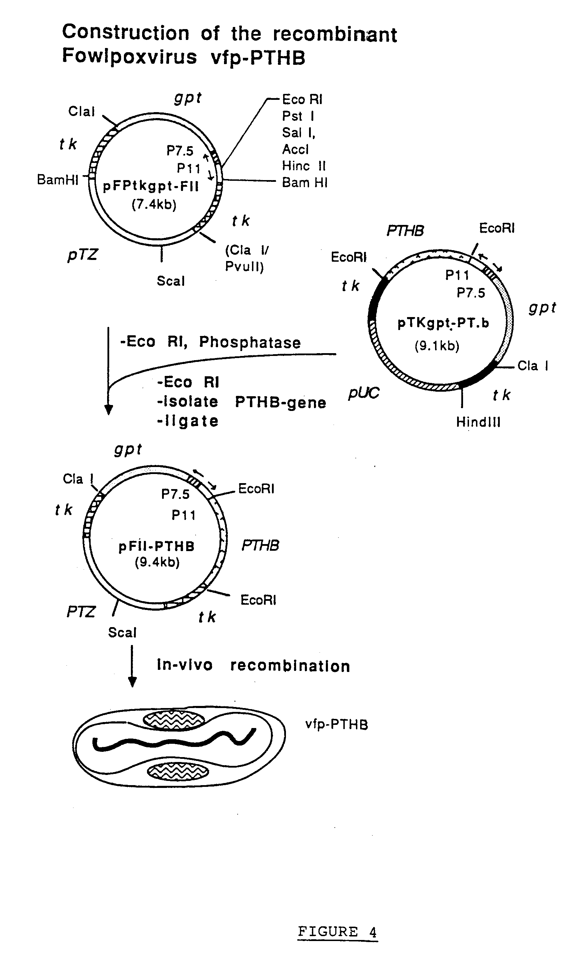

As shown in Figure 4, to construct the recombinant virus vfp-PTHB, the plasmid pTKgpt-PT.b, which contains the prothrombin cDNA as a 2.0 kb EcoRI fragment was cleaved with EcoRI. The prothrombin gene was purified out of low melting agarose as a 2.0 kb fragment and cloned into the unique Eco RI site of the fowlpox insertion plasmid pFPtkgpt-FII resulting in the plasmid pFII-PTHB. In this construct the coding sequence of the prothrombin gene is inserted in-frame behind the translational initiation codon of the vaccinia virus P11 promoter.

The plasmid pFII-PTHB was used to construct the fowlpox virus recombinant vfp-PTHB generally following the procedures described above in Example 9.

### Example 11

### Infection of susceptible avian cells with avipox virus recombinants.

After identification of the avipox virus recombinants two or more successive plaque purifications were performed in order to produce a pure recombinant virus. Susceptible cells, such as primary chicken embryo fibroblasts or other primary avian cells, were then infected in order to produce large strain solutions with recombinant viruses. The titre of this strain solution was determined by serial dilution and plaque tests.

In order to detect the expression of a foreign gene, the cells were infected with 1-20 pfu unpurified or purified virus per cell and incubated for 72-96 hours at 37^{o}C. The foreign gene product could be detected in the cell culture medium or within the cells depending on its nature. The products which remained within the cell were liberated by one or more of the following methods: subjection to ultrasound, cycles of freezing and thawing, homogenisation or detergent treatment.

The foreign protein was detected by immunological, enzymatic or electrophoretic methods and harvested by the usual techniques known in the art.

### Example 12

### Infection of susceptible mammalian cells with avipox virus recombinants.

After identification of the avipox virus recombinants two or more successive plaque purifications were performed in order to produce a pure recombinant virus. Susceptible cells, such as Vero or CV-1 cells or human MRC-5 cells were then infected in order to test for synthesis of the foreign gene product of interest. Detection of the foreign gene product and harvesting the cells were carried out as for Example 11 above.

## Claims

1. Plasmid comprising a foreign DNA sequence coding for a proteinaceous material, at least one replicon (ori1), at least one selection marker (sm1), cloning sites (cs), and at least one promoter (pr1), in which the promoter and the cloning sites are flanked by DNA sequences of an avipox virus, and in which the foreign DNA is inserted into a cloning site immediately after an ATG initiation codon supplied by the promoter or after one or two purine residues following the ATG codon.

2. Plasmid according to claim 1, wherein the cloning site (cs) is a multiple cloning site (mcs).

3. Plasmid according to claim 1 or 2, wherein the cloning site has one of the following sequences:
a) ATG AAT TCC TGC AGG TCG ACT CTA GAG GAT CCC CTT AAG TTA ACT TAA;
b) ATG GAA TTC CTG CAG GTC GAC TCT AGA GGA TCC CCT TAA GTT AAC TTA A;
c) ATG GGA ATT CCT GCA GGT CGA CTC TAG AGG ATC CCC TTA AGT TAA CTT AA.

4. Plasmid according to claims 1 to 3, wherein the promoter (pr1) is a promoter from poxvirus.

5. Plasmid according to claim 4, wherein the the promoter is the vaccinia promoter P11.

6. Plasmid according to claim 4, wherein the promoter is the late fowlpox promoter P25.

7. Plasmid according to claims 1 to 6, wherein the flanking DNA sequences of the avipox virus correspond to non-essential regions of the virus genome.

8. Plasmid according to claims 1 to 7, wherein the plasmid additionally carries a replicon (ori2) which permits the production of single-stranded DNA.

9. Plasmid according to claims 1 to 8, wherein the plasmid contains a second selection marker (sm2).

10. Plasmid according to claim 9, wherein the second selection marker (sm2) comprises DNA coding for xanthine-guanine-phosphoribosyl-transferase or DNA coding for a protein inactivating the antibiotic hygromycin.

11. Plasmid according to claim 10, wherein the second selection marker (sm2) comprises DNA corresponding to the lacZ gene of E. coli.

12. Recombinant avipox virus expression vector obtainable by in vivo homologous recombination of wild-type avipox virus with a plasmid according to claims 1 to 11.

13. Recombinant avipox virus expression vector , wherein the avipox virus is a fowlpox virus.

14. Recombinant avipox virus expression vector according to claims 12 or 13, wherein the vector contains, in addition to natural DNA of avipox virus, an additional promoter which controls the expression of the foreign DNA.

15. A mammalian cell culture comprising cells containing a recombinant avipox virus expression vector according to claims 12 and 13, said cell culture expressing foreign DNA coding for a proteinaceous material.

16. A primary chicken embryo fibroblast cell culture comprising cells containing a recombinant avipox expression vector according to claims 12 to 14, said cell culture expressing foreign DNA coding for a proteinaceous material.

17. A process for the preparation of a proteinaceous material, said process comprising growing animal cells containing a recombinant avipox virus expression vector according to claims 12 to 14, said cells expressing foreign DNA coding for the proteinaceous material and harvesting said proteinaceous material.

18. The process according to claim 17, in which the proteinaceous material is a vitamin K-dependent blood factor.

19. The process as claimed in claim 17, wherein the inserted DNA corresponds wholly or partially to the coding sequences for proteins or derivatives therefore from pathogens which can cause the following illnesses: pertussis, tetanus, malaria, AIDS, tick-borne encephalitis, hepatitis B, herpes infections and the poultry diseases M.rek's disease, ILT, infectious bronchitis or coccidiosis.

20. The process as claimed in anyone of claims 17 to 19, wherein the inserted foreigen DNA codes wholly or partially for a protein with the biological function of at least one of the following proteins: one of the blood clotting factors VIII, IX, XII, XIII or prtoein S, von Willebrand factor, protein C, prothrombin, antithrombin III, plasminogen, hirudin, NGF, FGF, TNF, B cell stimulating factors, proteins from the interleukin group, apolipoproteins or glycoproteins from viruses.

## Patentansprüche

1. Plasmid, umfassend eine fremde DNA-Sequenz, die für ein proteinhaltiges Material codiert, mindestens ein Replikon (ori1), mindestens einen Selektionsmarker (sm1), Clonierungsstellen (cs) und mindestens einen Promotor (pr1), wobei der Promotor und die Clonierungsstellen von DNA-Sequenzen eines Avipox-Virus flankiert werden und wobei die fremde DNA in eine Clonierungsstelle unmittelbar nach einem ATG-Initiationscodon, das von dem Promotor bereitgestellt wird, oder nach einem oder zwei Purin-Resten, die dem ATG-Codon folgen, inseriert ist.

2. Plasmid nach Anspruch 1, wobei die Clonierungsstelle (cs) eine mehrfache Clonierungsstelle (mcs) ist.

3. Plasmid nach Anspruch 1 oder 2, wobei die Clonierungsstelle eine der nachstehenden Sequenzen aufweist:
a) ATG AAT TCC TGC AGG TCG ACT CTA GAG GAT CCC CTT AAG TTA ACT TAA;
b) ATG GAA TTC CTG CAG GTC GAC TCT AGA GGA TCC CCT TAA GTT AAC TTA A;
c) ATG GGA ATT CCT GCA GGT CGA CTC TAG AGG ATC CCC TTA AGT TAA CTT AA.

4. Plasmid nach einem der Ansprüche 1 bis 3, wobei der Promotor (pr1) ein Promotor aus einem Pocken-Virus ist.

5. Plasmid nach Anspruch 4, wobei der Promotor der Vaccinia-Promotor P11 ist.

6. Plasmid nach Anspruch 4, wobei der Promotor der späte Fowlpox-Promotor P25 ist.

7. Plasmid nach einem der Ansprüche 1 bis 6, wobei die flankierenden DNA-Sequenzen des Avipox-Virus nicht-essentiellen Regionen des Virusgenoms entsprechen.

8. Plasmid nach einem der Ansprüche 1 bis 7, wobei das Plasmid zusätzlich ein Replikon (ori2) trägt, das die Bildung einzelsträngiger DNA erlaubt.

9. Plasmid nach einem der Ansprüche 1 bis 8, wobei das Plasmid einen zweiten Selektionsmarker (sm2) enthält.

10. Plasmid nach Anspruch 9, wobei der zweite Selektionsmarker (sm2) DNA umfaßt, die für Xanthin-Guanin-Phosphoribosyltransferase codiert, oder DNA, die für ein Protein codiert, das das Antibiotikum Hygromycin inaktiviert.

11. Plasmid nach Anspruch 10, wobei der zweite Selektionsmarker (sm2) DNA umfaßt, die dem lacZ-Gen von E. coli entspricht.

12. Rekombinanter Avipox-Virusexpressionsvektor, der durch in vivo erfolgende homologe Rekombination des Wildtyp-Avipox-Virus mit einem Plasmid nach einem der Ansprüche 1 bis 11 erhältlich ist.

13. Rekombinanter Avipox-Virusexpressionsvektor, wobei es sich bei dem Avipox-Virus um einen Fowlpox-Virus handelt.

14. Rekombinanter Avipox-Virusexpressionsvektor nach einem der Ansprüche 12 oder 13, wobei der Vektor zusätzlich zur natürlichen DNA des Avipox-Virus einen weiteren Promotor enthält, der die Expression der fremden DNA steuert.

15. Säugerzellkultur, umfassend Zellen, die einen rekombinanten Avipox-Virusexpressionsvektor gemäß den Ansprüchen 12 und 13 enthalten, wobei die Zellkultur fremde DNA, die für ein proteinhaltiges Material codiert, exprimiert.

16. Primäre Hühnerembryofibroblasten-Zellkultur, umfassend Zellen, die einen rekombinanten Avipox-Expressionsvektor gemäß einem der Ansprüche 12 bis 14 enthalten, wobei die Zellkultur fremde DNA, die für ein proteinhaltiges Material codiert, exprimiert.

17. Verfahren zur Herstellung eines proteinhaltigen Materials, wobei das Verfahren das Züchten von tierischen Zellen, die einen rekombinanten Avipox-Virusexpressionsvektor nach einem der Ansprüche 12 bis 14 enthalten, wobei die Zellen die fremde DNA, die für das proteinhaltige Material codiert, exprimieren, und die Gewinnung dieses proteinhaltigen Materials umfaßt.

18. Verfahren nach Anspruch 17, wobei es sich bei dem proteinhaltigen Material um einen von Vitamin K abhängigen Blutfaktor handelt.

19. Verfahren nach Anspruch 17, wobei die inserierte DNA ganz oder teilweise den codierenden Sequenzen für Proteine oder Derivate davon von Pathogenen entspricht, die die nachstehenden Erkrankungen verursachen können: Pertussis, Tetanus, Malaria, AIDS, von Zecken übertragene Encephalitis, Hepatitis B, Herpes-Infektionen und die Geflügelkrankheiten Mareksche Krankheit, ILT, infektiöse Bronchitis oder Coccidiose.

20. Verfahren nach einem der Ansprüche 17 bis 19, wobei die inserierte fremde DNA ganz oder teilweise für ein Protein mit der biologischen Funktion mindestens eines der nachstehenden Proteine codiert: einer der Blutgerinnungsfaktoren VIII, IX, XII, XIII oder Protein S, von Willebrand-Faktor, Protein C, Prothrombin, Antithrombin III, Plasminogen, Hirudin, NGF, FGF, TNF, B-Zellstimulierende Faktoren, Proteine aus der Interleukingruppe, Apolipoproteine oder Glycoproteine von Viren.

## Revendications

1. Plasmide comprenant un séquence d'ADN étranger codant pour une matière protéinique, au moins un réplicon (ori1), au moins un marqueur de sélection (sm1), des sites de clonage (cs), et au moins un promoteur (pr1), dans lequel le promoteur et les sites de clonage sont encadrés par des séquences d'ADN d'un virus avipox, et dans lequel l'ADN étranger est inséré dans un site de clonage aussitôt après un codon d'initiation ATG fourni par le promoteur ou après un ou deux radicaux purine faisant suite au codon ATG.

2. Plasmide selon la revendication 1, dans lequel le site de clonage (cs) est un site de clonage multiple (mcs).

3. Plasmide selon les revendications 1 ou 2, dans lequel le site de clonage possède une des séquences suivantes :
a) ATG AAT TCC TGC AGG TCG ACT CTA GAG GAT CCC CTT AAG TTA ACT TAA;
b) ATG GAA TTC CTG CAG GTC GAC TCT AGA GGA TCC CCT TAA GTT AAC TTA A;
c) ATG GGA ATT CCT GCA GGT CGA CTC TAG AGG ATC CCC TTA AGT TAA CTT AA.

4. Plasmide selon les revendications 1 à 3, dans lequel le promoteur (pr1) est un promoteur de poxvirus.

5. Plasmide selon la revendication 4, dans lequel le promoteur est le promoteur P11 de la vaccine.

6. Plasmide selon la revendication 4, dans lequel le promoteur est le promoteur P25 tardif de la variole aviaire.

7. Plasmide selon les revendication 1 à 6, dans lequel les séquences d'ADN adjacentes du virus avipox correspondent à des régions non essentielles du génome du virus.

8. Plasmide selon les revendications 1 à 7, dans lequel le plasmide porte en outre un réplicon (ori2) qui permet la production d'un ADN à simple brin.

9. Plasmide selon les revendications 1 à 8, dans lequel le plasmide contient un second marqueur de sélection (sm2).

10. Plasmide selon la revendication 9, dans lequel le second marqueur de sélection (sm2) comprend un ADN codant pour la xanthine-guanidine-phosphoribosyltransférase ou un ADN codant pour une protéine inactivant l'antibiotique hygromycine.

11. Plasmide selon la revendication 10, dans lequel le second marqueur de sélection (sm2) comprend un ADN correspondant au gène lacZ de E. coli.

12. Vecteur d'expression du virus avipox recombiné pouvant être obtenu par une recombinaison homologue in vivo du virus avipox du type sauvage avec un plasmide selon les revendications 1 à 11.

13. Vecteur d'expression du virus avipox recombiné, dans lequel le virus avipox est un virus de la variole aviaire.

14. Vecteur d'expression du virus avipox recombiné selon les revendications 12 ou 13, dans lequel le vecteur contient, en plus de l'ADN naturel du virus avipox, un promoteur supplémentaire qui commande l'expression de l'ADN étranger.

15. Culture de cellules de mammifères comprenant des cellules contenant un vecteur d'expression du virus avipox recombiné selon les revendica-tions 12 et 13, cette culture cellulaire exprimant l'ADN étranger codant pour une matière protéinique.

16. Culture de fibroblastes primaires d'embryons de poulets comprenant des cellules contenant un vecteur d'expression de l'avipox recombiné selon les revendications 12 à 14, cette culture cellulaire exprimant un ADN étranger codant pour une matière protéinique.

17. Procédé pour la préparation d'une matière protéinique, ce procédé comprenant le fait de cultiver des cellules animales contenant un vecteur d'expression du virus avipox recombiné selon les revendications 12 à 14, ces cellules exprimant un ADN étranger codant pour la matière protéinique et le fait de récolter cette matière protéinique.

18. Procédé selon la revendication 17, dans lequel la matière protéinique est un facteur sanguin dépendant de la vitamine K.

19. Procédé selon la revendication 17, dans lequel l'ADN inséré correspond en tout ou partie aux séquences de codage pour les protéines ou des dérivés de celles-ci provenant d'agents pathogènes qui peuvent causer les maladies suivantes : coqueluche, tétanos, malaria, SIDA, encéphalite due aux tiques, hépatite B, infections herpétiques et des maladies de la volaille telles que la maladie de Marek, l'ILT, la bronchite infectieuse ou la coccidiose.

20. Procédé selon l'une quelconque des revendications 17 à 19, dans lequel l'ADN étranger inséré code en tout ou partie pour une protéine ayant la fonction biologique d'au moins une des protéines suivantes : un des facteurs de coagulation du sang VIII, IX, XII, XIII ou protéine S, facteur de von Willebrand, protéine C, prothrombine, antithrombine III, plasminogène, hirudine, NGF, FGF, TNF, facteurs stimulant les cellules B, protéines du groupe de l'interleukine, apolipoprotéines ou glycoprotéines de virus.
